# EUROPEAN PATENT SPECIFICATION

(11) **EP 3 744 308 B1**
(45) Date of publication and mention of the grant of the patent: **27.12.2023**
(21) Application number: 18902999.4
(22) Date of filing: 28.02.2018
(51) Int. Cl.: A61B 17/43, A01N 1/02, A61B 10/00, A61D 19/02

(54) **SPERM COLLECTING CONTAINER**
SAMMELBEHÄLTER FÜR SPERMIEN
RÉCIPIENT DE COLLECTE DE SPERME

(30) Priority: 23.01.2018 JP 2018008713
(43) Date of publication of application: 02.12.2020
(73) Proprietor: Mitsuboshi Product Planning Co., Ltd., Tokyo 135-0063 (JP)
(72) Inventor: OKADA, Hiroshi, Koshigaya-shi, Saitama 343-0817 (JP); MUKAI, Toru, Tokyo 135-0063 (JP); AKIMOTO, Ryoji, Kawaguchi-shi, Saitama 332-0021 (JP)
(74) Representative: Mathys & Squire
(86) International application number: PCT/JP2018/007444
(87) International publication number: WO 2019/146128

(56) References cited:
- JP-A- 2006 305 237
- JP-A- 2012 115 829
- US-A- 4 312 350
- US-A- 6 050 935
- US-A1- 2006 228 794
- US-A1- 2009 004 058
- US-A1- 2012 078 136

## Description

### TECHNICAL FIELD

This disclosure relates to a sperm collection container.

### BACKGROUND ART

Conventionally, in assisted reproductive technology (ART) such as artificial insemination and in vitro fertilization, a patient collects his sperm in a transparent plastic sperm collection container (*i.e.,* container for collecting and storing sperm) having a cylindrical shape with a wide input port, and the patient brings the sperm collection container from home to a medical facility. In the medical facility, the semen brought by the patient is stored in the sperm collection container until a sperm inspection or a predetermined treatment is carried out.

With the conventional sperm collection container, the longer the carrying time and storage time of the collected sperm, the more it is affected by temperature change and oxidation due to contact with an air inside the sperm collection container, resulting in aging of the sperm. This affects the accuracy of fertilization and implantation in assisted reproductive technology.

Additionally, semen is viscous, and the conventional cylindrical sperm collection container has a too large bottom area to collect a several ml of sample. Therefore, the semen may diffuse in the container or may be attached to the wall surface of the container due to vibration and/or inclination during transportation, such that it may be difficult to sufficiently retrieve the semen from the container.

Another sperm collection container has been disclosed (see Patent Literature 1).

This sperm collection container can be folded into a flat state and can be used by expanding it into a cylindrical shape for collecting sperm. However, the object of the sperm collection container of Patent Literature 1 is to provide compact packaging in the unused state. That is, the object of the sperm collection container of Patent Literature 1 is not to make the sperm collection container compact during transportation of and/or storing of the sperm. Further, Patent Literature 1 is silent on preventing sperm deterioration.

US 2009/004058 A1 relates to an apparatus for an assay, and more particularly to an apparatus comprising a lateral flow assay test strip. The apparatus provides for detection of an analyte in a biological sample, with minimal handling and/or manipulation of the sample.

### CITATION LIST

### Patent Literature

Patent Literature 1: JPH08(1996)-299347 A

### SUM MARY

### Technical Problem

In view of the above problem, this disclosure provides a sperm collection container having an excellent effect of suppressing sperm deterioration and being capable of retrieving sperm efficiently.

### Solution to Problem

In order to achieve the above object, a sperm collection container according to claim 1 is disclosed.

### Advantageous Effects

With this disclosure, it is possible to provide a sperm collection container which has an excellent effect of suppressing sperm deterioration and is capable of retrieving sperm efficiently.

### BRIEF DESCRIPTION OF DRAWINGS

FIG. 1A is a front view of a sperm collection container according to a first embodiment.
FIG. 1B is an exploded front view of the sperm collection container according to the first embodiment.
FIG. 2 is a view showing a container body shown in FIG. 1A and FIG. 1B; in which a plan view is illustrated in the top left of the paper, a front view is illustrated in the left center, a bottom view is illustrated in the bottom left, and a cross-sectional view taken along the line A-A' in the plan view is illustrated in the right of the paper together with an enlarged cross-sectional view near a retrieval part thereof.
FIG. 3 is a view showing a lib body of a lid shown in FIG. 1; in which a plan view is illustrated in the top left of the paper, a front view is illustrated in the left center of the paper, a bottom view is illustrated in the bottom left of the paper, and a cross-sectional view taken along the line B-B' in the plan view is illustrated in the right of the paper.
FIG. 4 is a view for explaining a sperm collection procedure with the sperm collection container of the first embodiment.
FIG. 5 is a view for explaining a sperm retrieving procedure from the sperm collection container of the first embodiment.
FIG. 6A is a view showing a container body in a first variation, particularly an enlarged cross-sectional view near a retrieval part of the container body in the first variation in which a film is attached to an inner surface of a retrieving port.
FIG. 6B is a view showing a container body in a second variation, particularly an enlarged cross-sectional view near a retrieval part of the container body in the second variation in which a film is attached to an outer surface of a retrieving port.
FIG. 7A is a view showing a lid in a third variation, particularly a cross-sectional view of the lid in the third variation in which a supply port for inert gas is provided.
FIG. 7B is a view showing a lid in a fourth variation, particularly a cross-sectional view of the solid lid in the fourth variation.
FIG. 7C is a view showing a lid in a fifth variation, particularly a cross-sectional view of the solid lid in the fifth variation in which a supply port for inert gas is provided.

### DESCRIPTION OF EMBODIMENT

### First Embodiment

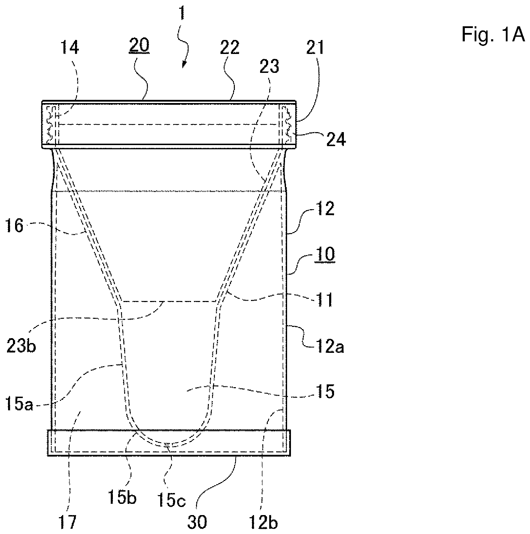

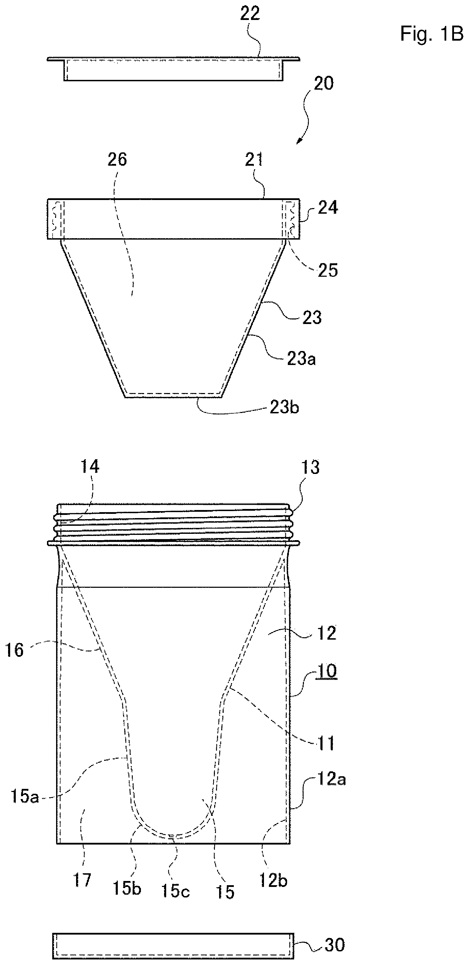

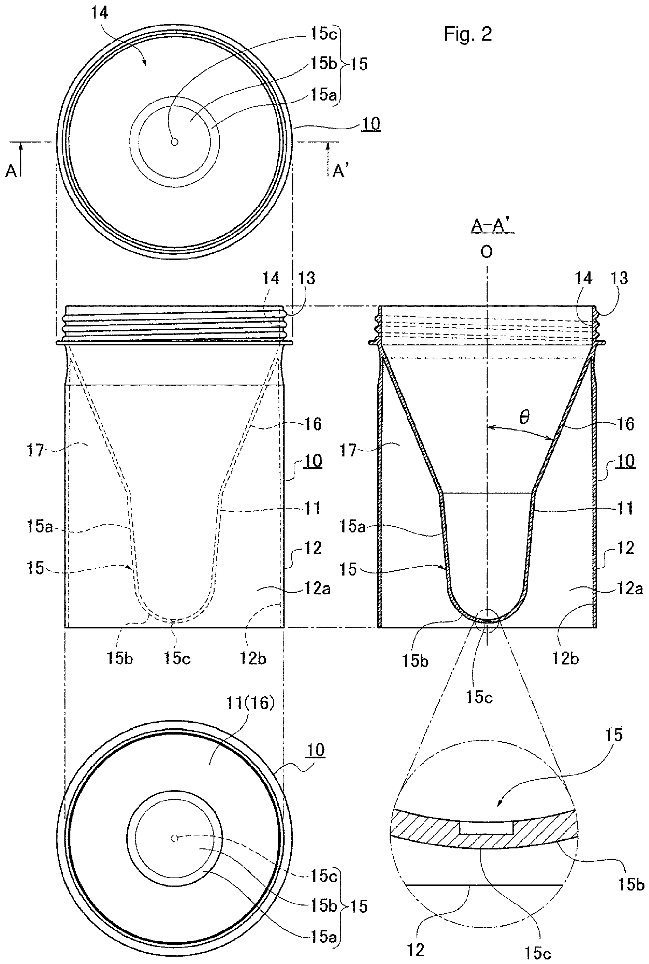

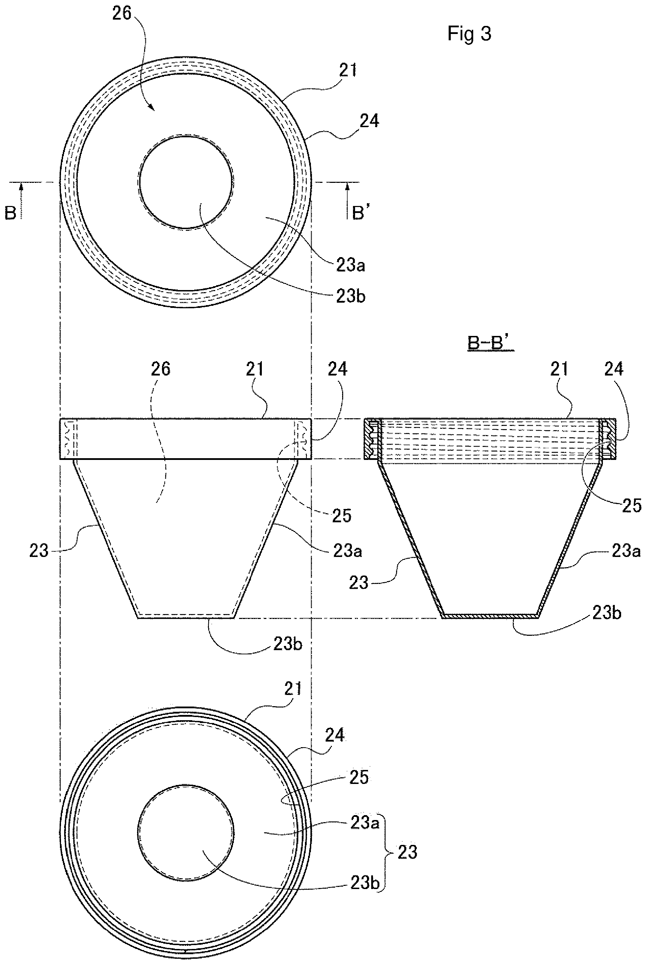

Hereinafter, a sperm collection container in a first embodiment according to this disclosure will be described with reference to FIG. 1A to FIG. 3. FIG. 1A is a front view of a sperm collection container 1 according to a first embodiment. FIG. 1B is an exploded front view of the sperm collection container 1. FIG. 2 shows a plan view, a front view, a bottom view, and a cross-sectional view of a container body 10 taken along the line A-A' in the plan view together with an enlarged cross-sectional view near a retrieval part thereof. FIG. 3 shows a plan view, a front view, a bottom view, and a cross-sectional view of a lid body 21 of a lid 20 taken along the line B-B' in the plan view.

A user of the sperm collection container 1 of this embodiment would be a patient who takes an ART, a doctor, or a health care worker such as an ART embryologist. For example, a patient (male) collects his sperm with the sperm collection container 1 of this embodiment at home, and then the patient himself or his wife brings the sperm collection container 1 to a medical facility. Alternatively, sperm may be collected at a medical facility. In the medical facility, the collected sperm is stored in the sperm collection container 1 until a doctor or the like carries out a sperm inspection or various treatments for artificial insemination. The sperm collection container 1 of this embodiment is used to collect sperm efficiently and to suppress sperm deterioration caused by oxidation, temperature change, and/or aging from the time of the sperm collection to a treatment for ART.

As shown in FIG. 1A and FIG. 1B, the sperm collection container 1 of the first embodiment includes a container body 10 for collecting sperm (semen), a lid 20 to be attached to an opening of the container body 10, and a bottom lid 30 to be attached to the bottom of the container body 10.

The container body 10 has a double structure of an inner container 11 with a bottom to store the collected sperm and an outer container 12 to cover the inner container 11. The inner container 11 and the outer container 12 are integrally connected to each other at the top thereof. The outer circumferential part of the connection is provided with a thread groove 13 to be attached with the lid 20.

As shown in the views of FIG. 2, the inner container 11 includes an input port 14 for inputting sperm and a storage portion 15 for storing the sperm. The input port 14 has a larger diameter than the storage portion 15, and the input port 14 and the storage portion 15 are connected via a taper portion 16 having a truncated cone shape (*i.e.,* funnel-shape) that gradually decreases in diameter toward the storage portion 15.

The input port 14 has a large circular shape in plan view in order to facilitate sperm collection. The inner diameter of the storage portion 15 is smaller than the inner diameter of the input port 14. The storage portion 15 includes a wall part 15a and a bottom part 15b that is protruded downward with an arc shape in cross section from the lower end of the wall part 15a. As shown in the views of FIG. 2, the storage portion 15 in this embodiment has the truncated cone shape in order to facilitate removal of the storage portion 15 from a formwork and to facilitate sperm collection.

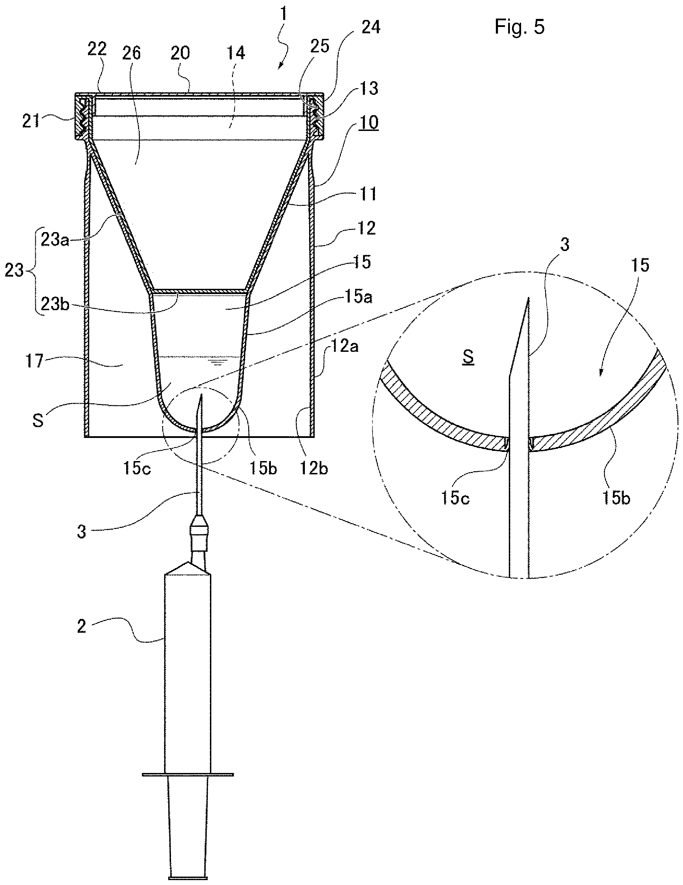

The bottom part 15b is provided with a retrieval part 15c through which the sperm stored in the storage portion 15 is retrieved with a retrieving means such as a syringe 2 (*see* FIG. 5). In this embodiment, a circular thin portion is formed in the vicinity of the center of the bottom part 15b and the thin portion corresponds to the retrieval part 15c, as shown in the enlarged view on the right of the paper in FIG. 2. The retrieval part 15c can be easily pierced by a piercing means, for example, a tip of the syringe 2 or a tip of a needle 3 (*see* FIG. 5) attached to the syringe 2, such that the retrieval part 15c is opened.

In this embodiment, the whole retrieval part 15c is thinned. However, it is not limited thereto. For example, only the outline of the circular retrieval part 15c may be thinned. Even in such a case, it is possible to open the retrieval part 15c by easily breaking the thin portion with a tip of the syringe 2 or with the needle 3.

As the amount of sperm (semen) collected once is 3 to 10 cc, the sperm diffuses and the sperm layer becomes thin, or the sperm adheres to a wall surface in the conventional sperm collection container having the same inner diameter from the input port to the storage portion. Therefore, it is difficult to retrieve the sperm from the conventional container and is hard to determine the amount of the collected sperm with the conventional container. In contrary, the storage portion 15 of the inner container 11 of this embodiment has a diameter smaller than that of the input port 14. With this, the sperm layer in the storage portion 15 becomes thick. Therefore, it is easy to retrieve the sperm with the syringe 2 or the like and is able to precisely measure the amount of the collected sperm.

The taper portion 16 collects the sperm inputted through the input port 14 and guides the sperm to the storage portion 15. In this embodiment, the inner wall surface of the taper portion 16 is mirror-finished. Accordingly, it improves the flow of sperm and to prevent the sperm from adhering to the inner wall surface, such that the sperm is guided to the storage portion 15 smoothly. It should be noted that the inner wall surface of the storage portion 15 may also be mirror-finished to prevent the sperm from adhering to the inner wall surface of the storage portion 15.

The outer container 12 is provided to protect the inner container 11. Thus, the outer container 12 is able to cover the entire inner container 11 and has a cylindrical outer peripheral wall 12a without a bottom. The bottom lid 30 is detachably attached to an opening 12b at the lower end of the outer peripheral wall 12a.

By attaching the bottom lid 30 to the outer container 12, the protection performance of the retrieval part 15c improves. As a result, it prevents the retrieval part 15c from being accidentally broken or the like due to an article in a bag hitting the retrieval part 15c. Further, by attaching the bottom lid 30 to the outer container 12, a space 17 partitioned by the outer peripheral wall 12a of the outer container 12, the bottom lid 30, and the inner container having the truncated cone shape is formed, and the air layer formed in the space 17 functions as a thermal insulator. Due to the thermal insulation effect of the air layer, the sperm inside the storage portion 15 is not unnecessarily influenced by the outside air temperature, thereby it is possible to suppress a rapid temperature change and to keep a predetermined temperature. Further, an insulating polystyrene foam or the like may be installed in the space 17 such that vibration and/or impact on the inner container 11 during transportation can be reduced.

On the other hand, by detaching the bottom lid 30, the sperm can be retrieved through the retrieval part 15c. As the sperm can be collected without removing the lid 20, it can prevent the sperm from contacting the outside air, resulting in that oxidation of the sperm and dust intrusion can be effectively prevented.

It should be noted that the storage temperature of sperm is preferably 20 to 35°C, and the most preferable temperature is about 25°C such that it is possible to improve the effect of suppressing deterioration of sperm over time. Here, the average human body temperature is about 37°C, and the sperm temperature at the time of collection is about 35°C. By storing the sperm in the double-structured sperm collection container 1 of this embodiment, it is possible to prevent a rapid temperature change or the like of the sperm and to keep the storage temperature of the sperm between 25°C, which is close to a room temperature, and 35°C, which is the temperature at the time of collection.

Although it is not limited, the container body 10 may preferably have the outer diameter of 50 to 100 mm and the height of 70 to 150 mm. This size makes the patient grab the container body 10 easily, thereby improving handleability during sperm collection and inspection. Additionally, it is possible to widen the input port 14 of the inner container 11 to facilitate sperm collection. Further, the sperm collection container 1 of this embodiment does not become bulky when stored in a bag or the like, thereby having a good portability. The container also improves stability when placed in a storage.

As illustrated in the cross-sectional view on the right of the paper in FIG. 2, the taper portion 16 has the truncated cone shape that is rotated about a rotation axis O. Here, it is preferable to have an inclined angle θ of the wall surface with respect to the rotation axis O 10° to 30° (*i.e.,* having apex angle of cone 20° to 60° and elevation angle of 60° to 80°). At the taper portion 16, sperm smoothly flows downward to the storage portion 15 along the inclined inner wall surface. Accordingly, it facilitates the sperm collection efficiently.

In this embodiment, the container body 10 has the height of about 83 mm and the outer diameter (*i.e.,* outer diameter of outer container 12) of about 57 mm. The inner diameter of the input port 14 is widened compared to that of the conventional container and is about 55 mm to facilitate the sperm collection. The storage portion 15 has the maximum inner diameter at the upper end of about 23 mm and has the height of about 34 mm. As a result, the storage portion 15 has the capacity of about 14 ml. The taper portion 16 has the height of about 38 mm and the inclined angle θ of about 23° (*i.e.,* elevation angle of about 67°).

Although it is not limited, a material of the container body 10 may preferably be a synthetic resin (*e*.*g*., plastic) such as polypropylene to have a light container body 10 with excellent impact resistance and water resistance. The container body 10 made of such a material may be transparent, translucent (semitransparent), or opaque.

In this embodiment, the container body 10 is produced by form molding from polypropylene such that the inner container 11 and the outer container 12 are integrally connected with each other. Accordingly, it is possible to produce the container body 10 easily at low cost.

In this embodiment, the outer surface of the container body 10 is applied with frosted glass processing to be translucent or semitransparent. This translucent container body 10 can hide the collected sperm from a third person, thereby reducing a mental burden on the patient. Further, by making the container body 10 translucent or semitransparent, the patient, a doctor or the like can visually inspect the amount and the state of the collected sperm in the storage portion 15 at a close range.

When the container body 10 is made opaque, the light shading property and the shielding property of the container body 10 improves. Therefore, it is possible to suppress the deterioration of the collected sperm due to ultraviolet rays and the like and has an excellent effect on hiding the sperm inside the storage portion 15 from a third person.

When the container body 10 is made transparent, it facilitates to inspect the amount and the state of the collected sperm. Even with the transparent container body 10, it is possible to hide the collected sperm from a third person by putting the sperm collection container 1 in a cloth bag, a paper bag, a storage container, or the like.

The lid 20 is attached to the opening of the above container body 10 to seal the container body 10. As illustrated in the views of FIG. 3, the lid 20 includes a lid body 21 detachably attached to the container body 10 and an upper lid 22 detachably attached to the lid body 21.

The lid body 21 is provided with an inner lid 23 configured to be inserted into the inner container 11. The inner lid 23 protrudes downward so as to reduce the capacity of the inner container 11. At the upper end of the lid body 21, a grip portion 24 is provided outside the inner lid 23 with a gap therebetween. The grip portion 24 is a portion which a patient or the like grips when the lid 20 is attached or detached. On the inner circumference of the grip portion 24, a thread groove 25 that is screwed into the thread groove 13 of the container body 10 is provided. Additionally, a packing or the like may be provided inside the grip portion 24 to further enhance the effect of preventing sperm leaking and outside air intrusion.

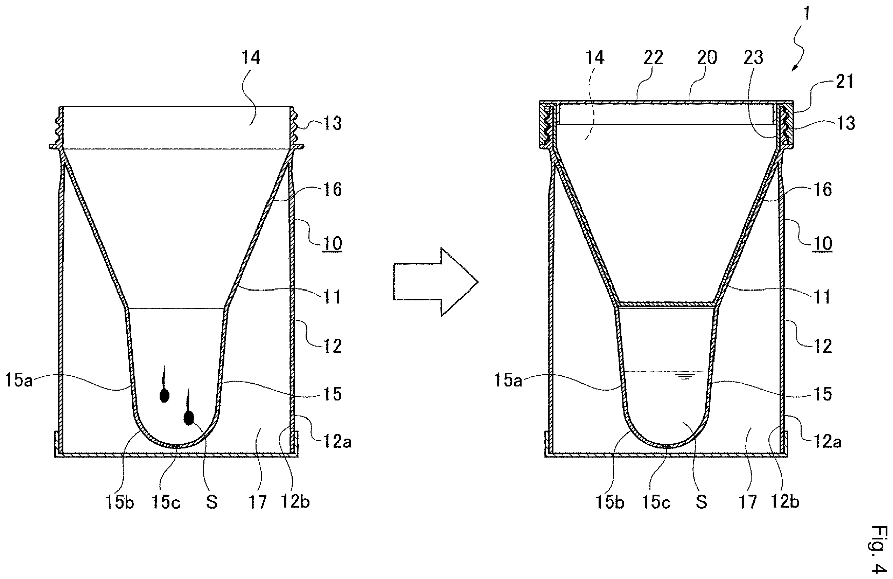

The inner lid 23 is formed as a hollow body and has a truncated cone shape protruding toward the storage portion 15 of the inner container 11 so as to correspond to the shape of the taper portion 16 of the inner container 11. That is, the inner lid 23 is inserted to and placed in the taper portion 16. The inner lid 23 includes a wall part 23a having the truncated cone shape, a flat bottom part 23b, and a space 26 surrounded by the wall part 23a and the bottom part 23b, so as to have the hollow body. By inserting the inner lid 23 into the taper portion 16, the volume of the air layer that contacts the sperm inside the storage portion 15 is made as small as possible (see drawing on right of FIG. 4).

On the upper side of the lid body 21, which is opposite to the side where the lid body 21 is inserted into the inner container 11, the upper lid 22 is detachably attached. By attaching the upper lid 22 to the lid body 21, the space 26 of the inner lid 23 is isolated from the outside. With the thermal insulation effect of the air layer in the space 26, it is possible to further improve the effect of suppressing the temperature change of the sperm in the storage portion 15.

As the upper lid 22 is detachably provided, it is also possible to put a polystyrene foam or the like into the space 26. Alternatively, it is possible to put a heat storage material, a heat generating material, a cold storage material or the like into the space 26 in accordance with the outside temperature or the like. With this, it is possible to freely control the temperature inside the container body 10 so as not to be affected by the outside temperature even when the outside temperature is excessively high or low.

An example of use of the sperm collection container 1 of the first embodiment will be described with reference to FIG. 4 and FIG. 5. FIG. 4 is a view for explaining a sperm collection procedure with the sperm collection container 1. FIG. 5 is a view for explaining a sperm retrieving procedure from the sperm collection container 1 of the first embodiment.

As illustrated in the left drawing of FIG. 4, a patient removes the lid 20 from the sperm collection container 1 to collect his sperm S with the container body 10. Through the wide-opened input port 14 of the inner container 11, the sperm S directly drops to the storage portion 15 or flows downward smoothly along the mirror-finished and inclined inner wall surface of the taper portion 16 to the storage portion 15 and then is stored therein.

After collection of the sperm S, the patient puts the lid 20 on the opening of the container body 10 and screws the thread groove 25 of the lid 20 and the thread groove 13 of the container body 10 to attach the lid 20 to the container body 10 (see right drawing of FIG. 4). Thus, the inner lid 23 is inserted to and placed in the taper portion 16 of the inner container 11, such that the volume of the air layer in the inner container 11 decreases. As a result, it is possible to minimize the contact between the sperm S and the air in the storage portion 15.

By putting the sperm collection container 1 with the collected sperm S into a bag or so, the patient can easily bring the sperm collection container 1 to a medical facility. In this case, the patient can put the sperm collection container 1 into a paper bag, a cloth bag, or a resin bag, etc. and then can put it into his bag to suppress visibility from a third person. Alternatively, it is possible to put the sperm collection container 1 into a dedicated container such as a cold storage bag or a bag having a thermal insulation material or a temperature adjusting device. This way, it can suppress the temperature change as well as facilitate the temperature management. At the medical facility, the sperm S can be stored in the sperm collection container 1 until a sperm inspection or a predetermined treatment is carried out.

When retrieving the sperm S from the sperm collection container 1 for an inspection or a treatment at the medical facility, the bottom lid 30 is removed from the lower end of the outer container 12 to open the opening 12b at the lower end of the outer container 12. Through the opening 12b, the needle 3 of the syringe 2 is pierced into the retrieval part 15c at the bottom part 15b, such that the retrieval part 15c is broken and the needle 3 is inserted into the storage portion 15, as illustrated in FIG. 5. The sperm S in the storage portion 15 is then sucked into the syringe 2 and is placed in an inspection device, a refining device, or the like from the syringe 2.

As described above, the sperm collection container 1 of this embodiment includes the container body 10 having the inner container 11 to store sperm and the outer container 12 to cover the inner container 11, and the lid 20 to be attached to the container body 10. The inner container 11 includes the input port 14 through which sperm is inputted and the bottomed storage portion 15 which has a diameter smaller than that of the input port 14 and stores the sperm. The bottom part 15b of the storage portion is provided with the retrieval part 15c. The retrieval part 15c is sealed when collecting the sperm and is opened by inserting a piercing member (*i.e.,* needle 3 or tip of syringe 2) therethrough when retrieving the sperm. The lid 20 includes the inner lid 23 configured to be inserted in and placed to the inner container 11 so as to reduce the capacity of the inner container 11.

With this, a patient can easily collect his sperm with the container body 10 having the wide-opened input port 14 while preventing liquid leakage. Further, the patient can easily grab the container body 10 since the outer container 12 has the cylindrical shape. Therefore, it is possible to reduce the patient's psychological resistance or anxiety of failure at the time of sperm collection. Additionally, since the inner diameter of the storage portion 15 is smaller than that of the input port 14 to reduce the capacity of the storage portion 15, it is possible to thicken the sperm layer. Therefore, it is possible to suppress diffusion of the collected sperm and to facilitate to determine the amount of the collected sperm or the like.

After the sperm collection, the lid 20 is attached to the container body 10 to sufficiently prevent outside air intrusion into the container body 10 and liquid leakage from the container body 10. Additionally, the volume of the air layer in the inner container 11 is reduced by the inner lid 23 in order to minimize the contact between the sperm S in the storage portion 15 and the air. As a result, it is possible to suppress sperm deterioration due to oxidation.

Since the container body 10 has the double structure with the inner container 11 and the outer container 12, the space 17 has the thermal insulation effect. With this, it is possible to suppress the influence of the outside air on the sperm in the storage portion 15 and to prevent a rapid temperature change or the like of the sperm. Therefore, it is possible to keep the storage temperature of the sperm at a predetermined temperature suitable for storage, for example around 25°C.

As it is possible to suppress the oxidation and the temperature change, the patient does not need to consider the storage temperature when bringing the container 1 to a medical facility and even does not need to hurry to bring the container 1 to a medical facility. Further, the sperm collected in the sperm collection container 1 does not diffuse even when the sperm collection container 1 is tilted, or so. This makes it easy for the patient to bring the sperm collection container 1 with a bag or so and can reduce mental pressure and urgency during transportation.

At the medical facility, it is possible to keep the sperm in the sperm collection container 1 as brought by the patient without strict temperature control. Therefore, it can reduce the labor or mental burden of the doctor and the like. Further the oxidation and the temperature change of the sperm is suppressed in the sperm collection container 1 of this embodiment, thereby preventing sperm deterioration due to aging.

In order to retrieve the sperm, the doctor or the like can easily retrieve the sperm from the storage portion 15 by piercing the thinned retrieval part 15c with the piercing means such as the needle 3. That is, the doctor or the like does not need to remove the lid 20. Therefore, it is possible to reliably prevent contact between the sperm and the outside air, as well as contamination with dust and the like during the retrieving procedure.

As described above, this embodiment can provide the sperm collection container 1 which has an excellent effect of suppressing sperm deterioration and is capable of retrieving sperm therefrom efficiently. As a result, it is possible to improve the accuracy of fertilization and implantation in assisted reproductive technology. Further, the sperm collection container 1 can be easily produced by form molding with resin or the like. Therefore, it is possible to mass-produce the sperm collection container 1, thereby it is possible to provide the sperm collection container 1 at low cost.

In this embodiment, the inner lid 23 includes the wall part 23a protruding toward inside the inner container 11, the bottom part 23b, and the space 26 surrounded by the wall part 23a and the bottom part 23b, so as to have the hollow body. The upper lid 22 to close the space 26 is detachably attached to the lid 20. With this, the air layer in the space 26 provides the thermal insulation effect and improves the effect of suppressing the temperature change of the sperm in the inner container 11. Additionally, it is also possible to further improve the effect of suppressing the temperature change by putting a thermal insulation material in the space 26. Alternatively, it is possible to put a heat storage material, a cold storage material or the like into the space 26 to freely control the temperature inside the inner container 11.

The outer container 12 is provided with the opening 12b through which the needle 3 corresponding to the piercing member is inserted, and the bottom lid 30 is detachably attached to the opening 12b. By attaching the bottom lid 30 to the opening 12b for the sperm collection or the transportation, it is possible to protect the retrieval part 15c and to enhance the thermal insulation effect. Further, it is possible to easily retrieve the sperm from the retrieval part 15c through the opening 12b by removing the bottom lid 30.

The input port 14 has a larger diameter than that of the storage portion 15, and the input port 14 and the storage portion 15 are connected via the taper portion 16 at which the diameter gradually decreases. The inner wall surface of the taper portion 16 is a mirror surface. With this, the sperm inputted through the input port 14 is gathered at the taper portion 16 and is smoothly guided to the storage portion 15. As a result, it is possible to prevent the sperm from adhering to the inner wall surface and to store the sperm in the storage portion 15 efficiently.

The taper portion 16 has the truncated cone shape, and the inclined angle of the wall surface with respect to the rotation axis is 10° to 30°. Therefore, the sperm inputted through the input port 14 is further smoothly guided to the storage portion 15. Additionally, the inner lid 23 has a supply port 27 to supply inert gas into the inner container 11. With this, it is possible to improve the effect of suppressing the sperm oxidation in the storage portion 15.

In this embodiment, the outer container 12 and the inner container 11 are made translucent or semitransparent. This makes it difficult for a third party to realize that the sperm is stored in the sperm collection container 1, such that it is possible to protect the patient privacy. On the other hand, it is possible to visually inspect the sperm in the inner container 11 at a close range, such that a doctor or the like can determine the amount and the state of the collected sperm. It should be noted that the above effect can be obtained even when only one of the outer container 12 and the inner container 11 is made translucent or semitransparent. Further, the retrieval part 15c of this embodiment is formed of the thin portion provided at the bottom part 15b of the storage portion 15. Accordingly, it is possible to prevent sperm leakage during sperm collection or storage, while it is possible to easily retrieve the sperm by piercing the thinned retrieval part 15c with the needle 3 or so.

### Variations

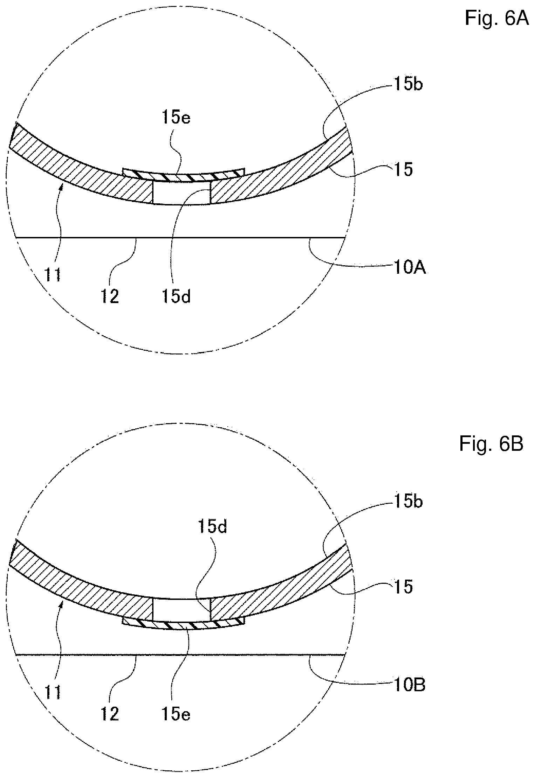

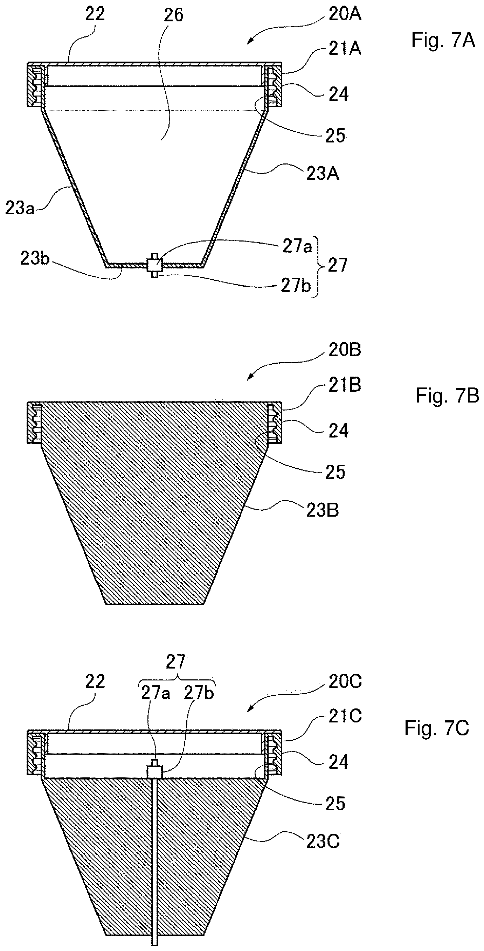

Hereinafter, variations of the container body 10 (first variation and second variation) and variations of the lid 20 (third to fifth variations) will be described with reference to FIG. 6A to FIG. 7C. FIG. 6A is an enlarged view near a retrieval part 15c of a container body 10A of the first variation. FIG. 6B is an enlarged view near a retrieval part 15c of a container body 10B of the second variation. FIG. 7A, FIG. 7B, and FIG. 7C are cross-sectional views of lids 20A, 20B, 20C of the third, fourth, and fifth variations.

In the container body 10A of the first variation shown in FIG. 6A, the retrieval part 15c includes a retrieving port 15d opened at a bottom part 15b of a storage portion 15 and a film 15e attached to an inner surface of the retrieving port 15d.

In the container body 10B of the second variation shown in FIG. 6B, the retrieval part 15c also includes a retrieving port 15d and a film 15e, but the film 15e in the second variation is attached to an outer surface of the retrieving port 15d.

The container bodies 10A, 10B of the first and second variations can be easily produced by molding the retrieving ports 15d with a mold having a projection and covering the retrieving ports 15d by the films 15e. Therefore, it is possible to mass-produce the container bodies 10A, 10B at low cost. At the time of sperm collection, the retrieving port 15d is covered and sealed by the film 15e so as to prevent sperm leakage from the storage portion 15. At the time of sperm retrieval, the film 15e is broken by the needle 3 or the like of the syringe 2 to open the retrieving port 15d, thereby the sperm in the storage portion 15 can be retrieved easily.

The lid 20A of the third variation shown in FIG. 7A is provided with a supply port 27, which supplies inert gas into the storage portion 15 of the inner container 11, at a bottom part 23b of an inner lid 23A of a lid body 21A. The supply port 27 includes a supply pipe 27a for supplying the inert gas and a check valve 27b that allows gas to flow into the inner container 11 but prevents gas from flowing out from the inner container 11 to the outside. It should be noted that the structure of the supply port 27 is not limited thereto. So long as it is capable of supplying inert gas into the inner container 11, the supply port 27 may include only the supply pipe 27a or the supply port 27 may be a hole opened at the bottom part 23b. These modifications are applicable to the fifth variation described later.

The inert gas is supplied to suppress the sperm oxidation due to oxygen in the air remaining in the storage portion 15. The preferable inert gas is, for example, argon gas (Ar), nitrogen gas (N2), carbon dioxide (CO2), or hydrogen (H2). It is possible to use these gases solely or as a mixed gas in which two or more kinds are mixed.

After sperm collection, the upper the lid 20A of the third variation is attached to the container body 10 of the first embodiment, to the container body 10A of the first variation, or to the container body 10B of the third variation, such that the air layer in the inner container 11 is minimized by the inner lid 23A. Then, the inert gas is supplied to the storage portion 15 from an inert gas source such as a gas cylinder through the supply port 27. Accordingly, the air remaining in the inner container 11 is exhausted to the outside from an appropriate exhaust mechanism, and the storage portion 15 is filled with the inert gas. As a result, it is possible to improve the effect of suppressing the sperm oxidation in the storage portion 15.

Next, the lid 20B of the fourth variation shown in FIG. 7B will be described. The inner lids 23, 23A of the first embodiment and the third variation are formed hollow, whereas the inner lid 23B of the lid 20B of the fourth variation is formed solid. The lid 20B can also be easily produced by molding with a synthetic resin material or the like. As the lid 20B does not need the upper lid 22, the inner lid 23B can be made more robust. Further, the thick synthetic resin material can achieve the thermal insulation effect, such that it is possible to suppress the temperature change of the sperm S.

In the fourth variation, the inner lid 23B is formed solid with the synthetic resin material. However, it is not limited thereto. For example, the inner lid 23B may be formed hollow, and the space 26 may be filled with an elastic member such as rubber and silicon or filled with a filling member such as a polystyrene foam. With this, it is possible to obtain a similar effect to a thermal insulation synthetic resin. Also, by filling with or removing the filing member, it is possible to select the solid inner lid 23B or the hollow inner lid 23B in accordance with the environmental conditions such as the outside temperature.

The lid 20C of the fifth variation shown in FIG. 7C has the characteristics of both third variation and the fourth variation. That is, the lid 20C of the fifth variation includes a solid inner lid 23C provided with a supply pipe 27a and a check valve 27b.

With the lid 20C, it is possible to further improve the effect of suppressing the sperm oxidation by supplying the inert gas into the storage portion 15 through the supply port 27. Additionally, it is possible to suppress the temperature change of the sperm due to the thermal insulation effect of the solid inner lid 23C.

Although the embodiment and the variations of the disclosure have been described in detail with reference to the drawings, the above embodiment and the variations are merely examples of the disclosure. The disclosure is not limited to the above-described embodiment and the variations. Any modifications made without departing from the gist of this disclosure are of course included in the scope of this disclosure.

For example, the outer container 12 of the above-described embodiment and the variations has the cylindrical shape. However, it is not limited thereto, and the shape may be prismatic, truncated cone, or truncated pyramid. The taper portion 16 and the storage portion 15 of the inner container 11 has the truncated cone shape. However, it is not limited thereto, and the shape may be a truncated pyramid. Alternatively, the taper portion 16 and the storage portion 15 may be formed into a cylindrical shape or a prismatic shape having different diameters from each other, and the inner container 11 may have a stepped shape in cross section. Additionally, it may be possible to provide a scale to the storage portion 15 so that the amount of the collected sperm can be measured. The inner container 11 should be made such that the input port 14 has a large opening and the storage portion 15 has a diameter smaller than that of the input port 14. That is, it is not necessary to connect the input port 14 and the storage portion 15 with the taper portion 16 or to make the inner wall surface have a tapered shape. Though, by providing the taper portion 16, it is possible to flow sperm downward smoothly to the storage portion 15.

In the above-described embodiment and the variations, the outer container 12 has the cylindrical shape without a bottom. However, as long as it is possible to retrieve the sperm from the retrieval part 15c, the outer container 12 may have a bottom. For example, such a bottom may be provided with a cutout or a hole through which the syringe 2 or the like can be inserted. Additionally, the bottom lid 30 may be detachably attached to the cutout or the hole to protect the retrieval part 15c.

In the above-described embodiment and the variations, the inner container 11 and the outer container 12 are integrally molded. However, it is not limited thereto. The inner container 11 and the outer container 12 may be molded separately, and the inner container 11 may be configured to be inserted into the outer container 12 to form the container body 10. In this case, only the inner container 11 may be disposable, and the outer container 12 may be reused.

### REFERENCE SIGNS LIST

1 Container body; 2 Syringe (Retrieving means; Piercing member); 3 Needle (Piercing member); 10, 10A, 10B Container body; 11 Inner container; 12 Outer container; 12a Outer peripheral wall; 12b Opening; 14 Input port; 15 Storage portion; 15b Bottom part; 15c Retrieval part; 15d Retrieving port; 15e Film; 16 Taper portion; 20, 20A, 20B, 20C Lid; 21, 21A, 21B, 21C Lid body; 22 Upper lid; 23, 23A, 23B, 23C Inner lid; 23a Wall part; 23b bottom part; 26 Space; 27 Supply port; 27a Supply pipe; 27b Check valve; 30 Bottom lid; S sperm

## Claims

1. A sperm collection container (1), comprises:
a container body (10) that comprises an inner container (11) configured to store sperm (S) and an outer container (12) configured to cover the inner container (11); and
a lid (20) that is configured to be attached to the container body (10), wherein
the inner container (11) comprises an input port (14) through which the sperm (S) is inputted and a bottomed storage portion (15) that is configured to store the sperm (S), the storage portion (15) having a diameter smaller than that of the input port (14),
the storage portion (15) comprises a retrieval part (15c) that is provided at a bottom part (15b) of the storage portion (15), the retrieval part (15c) being sealed when collecting the sperm (S) and being configured to be opened by inserting a piercing member (3) through the retrieval part (15c) when retrieving the sperm (S) from the storage portion (15),
the input port (14) and the storage portion (15) are connected via a taper portion (16) that gradually decreases in diameter toward the storage portion (15), and
the lid (20) comprises an inner lid (23) that is configured to be inserted into the inner container (11) so as to reduce capacity of the inner container (11), and
**characterized in that** the inner lid (23) is formed as a hollow body and has a truncated cone shape protruding toward the storage portion (15) of the inner container (11) so as to correspond to the shape of, and being configured to be inserted into, the taper portion (16) of the inner container (11)

2. The sperm collection container (1) according to claim 1, wherein the inner lid (23) comprises a wall part (23a) protruding toward the storage portion (15), a bottom part (23b), and a space (26) surrounded by the wall part (23a) and the bottom part (23b) so as to be formed as a hollow body, and
the lid (20) comprises an upper lid that is configured to be detachably attached to the lid (20) to close the space (26).

3. The sperm collection container (1) according to claims 1 or 2, wherein the outer container (12) is provided with an opening (12b) through which the piercing member (3) is inserted, and
the lid (20) comprises a bottom lid (30) that is configured to be detachably attached to the opening (12b).

4. The sperm collection container (1) according to any one of claims 1 to 3, wherein an inner wall surface of the taper portion (16) is a mirror surface.

5. The sperm collection container (1) according to any one of claims 1 to 3, wherein the inner lid (23) is provided with a supply port that is configured to supply inert gas into the inner container (11).

6. The sperm collection container (1) according to any one of claims 1 to 5, wherein at least one of the outer container (12) or the inner container (11) is made translucent.

7. The sperm collection container (1) according to any one of claims 1 to 6, wherein the retrieval part (15c) comprises a retrieving port (15d) opened at the bottom part (15b) of the storage portion (15) and a film (15e) attached to the retrieving port (15d).

8. The sperm collection container (1) according to any one of claims 1 to 6, wherein the retrieval part (15c) is formed of a thin portion provided at a part of the bottom part (15b) of the storage portion (15).

## Patentansprüche

1. Samenauffangbehälter (1), umfassend:
einen Behälterkörper (10), der einen inneren Behälter (11), der dazu konfiguriert ist, Samen (S) aufzubewahren, und einen äußeren Behälter (12), der dazu konfiguriert ist, den inneren Behälter (11) abzudecken, umfasst; und
einen Deckel (20), der dazu konfiguriert ist, an dem Behälterkörper (10) angebracht zu werden, wobei
der innere Behälter (11) eine Eingabeöffnung (14), durch die der Samen (S) eingegeben wird, und einen abgeflachten Aufbewahrungsabschnitt (15), der dazu konfiguriert ist, den Samen (S) aufzubewahren, umfasst, wobei der Aufbewahrungsabschnitt (15) einen Durchmesser aufweist, der kleiner als der der Eingabeöffnung (14) ist,
der Aufbewahrungsabschnitt (15) einen Rückgewinnungsteil (15c) umfasst, der an einem Bodenteil (15b) des Aufbewahrungsabschnitts (15) vorgesehen ist, wobei der Rückgewinnungsteil (15c) versiegelt ist, wenn der Samen (S) aufgefangen wird, und dazu konfiguriert ist, geöffnet zu werden, in dem ein Durchstechelement (3) durch den Rückgewinnungsteil (15c) eingeführt wird, wenn der Samen (S) aus dem Aufbewahrungsabschnitt (15) zurückgewonnen wird,
die Eingabeöffnung (14) und der Aufbewahrungsabschnitt (15) mittels eines Konusabschnitts (16) verbunden sind, dessen Durchmesser zu dem Aufbewahrungsabschnitt (15) hin allmählich abnimmt, und
der Deckel (20) einen inneren Deckel (23) umfasst, der dazu konfiguriert ist, in den inneren Behälter (11) eingeführt zu werden, um das Fassungsvermögen des inneren Behälters (11) zu verringern, und
**dadurch gekennzeichnet, dass** der innere Deckel (23) als ein Hohlkörper geformt ist und eine Kegelstumpfform aufweist, die zu dem Aufbewahrungsabschnitt (15) des inneren Behälters (11) hin vorsteht, um der Form des Konusabschnitts (16) des inneren Behälters (11) zu entsprechen und dazu konfiguriert zu sein, in diesen eingeführt zu werden.

2. Samenauffangbehälter (1) nach Anspruch 1, wobei der innere Deckel (23) einen Wandteil (23a), der zu dem Aufbewahrungsabschnitt (15) hin vorsteht, einen Bodenteil (23b) und einen Raum (26), der von dem Wandteil (23a) und dem Bodenteil (23b) umgeben ist, umfasst, um als ein Hohlkörper geformt zu sein, und
der Deckel (20) einen oberen Deckel umfasst, der dazu konfiguriert ist, abnehmbar an dem Deckel (20) angebracht zu werden, um den Raum (26) zu schließen.

3. Samenauffangbehälter (1) nach den Ansprüchen 1 oder 2, wobei der äußere Behälter (12) mit einer Öffnung (12b) versehen ist, durch die das Durchstechelement (3) eingeführt wird, und
der Deckel (20) einen unteren Deckel (30) umfasst, der dazu konfiguriert ist, abnehmbar an der Öffnung (12b) angebracht zu werden.

4. Samenauffangbehälter (1) nach einem der Ansprüche 1 bis 3, wobei eine Oberfläche der inneren Wand des Konusabschnitts (16) eine Spiegeloberfläche ist.

5. Samenauffangbehälter (1) nach einem der Ansprüche 1 bis 3, wobei der innere Deckel (23) mit einer Zufuhröffnung versehen ist, die dazu konfiguriert ist, Inertgas in den inneren Behälter (11) zuzuführen.

6. Samenauffangbehälter (1) nach einem der Ansprüche 1 bis 5, wobei mindestens einer von dem äußeren Behälter (12) oder dem inneren Behälter (11) durchscheinend hergestellt ist.

7. Samenauffangbehälter (1) nach einem der Ansprüche 1 bis 6, wobei der Rückgewinnungsteil (15c) eine Rückgewinnungsöffnung (15d), die am Bodenteil (15b) des Aufbewahrungsabschnitts (15) geöffnet wird, und eine Folie (15e), die an der Rückgewinnungsöffnung (15d) angebracht ist, umfasst.

8. Samenauffangbehälter (1) nach einem der Ansprüche 1 bis 6, wobei der Rückgewinnungsteil (15c) aus einem dünnen Abschnitt geformt ist, der an einem Teil des Bodenteils (15b) des Aufbewahrungsabschnitts (15) vorgesehen ist.

## Revendications

1. Contenant de collecte de sperme (1), comprenant :
un corps de contenant (10) qui comprend un contenant interne (11) configuré pour stocker du sperme (S) et un contenant externe (12) configuré pour couvrir le contenant interne (11) ; et
un couvercle (20) qui est configuré pour être attaché au corps de contenant (10), dans lequel
le contenant interne (11) comprend un orifice d'entrée (14) à travers lequel le sperme (S) est amené à entrer et une partie de stockage pourvue d'un fond (15) qui est configurée pour stocker le sperme (S), la partie de stockage (15) ayant un diamètre inférieur à celui de l'orifice d'entrée (14),
la partie de stockage (15) comprend une partie de récupération (15c) qui est fournie au niveau d'une partie inférieure (15b) de la partie de stockage (15), la partie de récupération (15c) étant fermée de manière étanche pendant la collecte du sperme (S) et étant configurée pour être ouverte par insertion d'un élément de perforation (3) à travers la partie de récupération (15c) pendant la collecte du sperme (S) depuis la partie de stockage (15),
l'orifice d'entrée (14) et la partie de stockage (15) sont connectés via une partie effilée (16) dont le diamètre diminue progressivement vers la partie de stockage (15), et
le couvercle (20) comprend un couvercle interne (23) qui est configuré pour être inséré jusque dans le contenant interne (11) de manière à réduire la capacité du contenant interne (11), et
**caractérisé en ce que** le couvercle interne (23) est formé comme un corps creux et a la forme d'un cône tronqué faisant saillie vers la partie de stockage (15) du contenant interne (11) de manière à correspondre à la forme de, et étant configuré pour être inséré jusque dans, la partie effilée (16) du contenant interne (11).

2. Contenant de collecte de sperme (1) selon la revendication 1, dans lequel le couvercle interne (23) comprend une partie de paroi (23a) faisant saillie vers la partie de stockage (15), une partie inférieure (23b), et
un espace (26) entouré par la partie de paroi (23a) et la partie inférieure (23b) de manière à être formé comme un corps creux, et le couvercle (20) comprend un couvercle supérieur qui est configuré pour être attaché de manière détachable au couvercle (20) pour former l'espace (26).

3. Contenant de collecte de sperme (1) selon la revendication 1 ou 2, dans lequel le contenant externe (12) est pourvu d'une ouverture (12b) à travers laquelle l'élément de perforation (3) est inséré, et
le couvercle (20) comprend un couvercle inférieur (30) qui est configuré pour être attaché de manière détachable à l'ouverture (12b).

4. Contenant de collecte de sperme (1) selon l'une quelconque des revendications 1 à 3, dans lequel une surface de paroi interne de la partie effilée (16) est une surface de miroir.

5. Contenant de collecte de sperme (1) selon l'une quelconque des revendications 1 à 3, dans lequel le couvercle interne (23) est pourvu d'un orifice d'alimentation qui est configuré pour alimenter le contenant interne (11) en gaz inerte.

6. Contenant de collecte de sperme (1) selon l'une quelconque des revendications 1 à 5, dans lequel au moins l'un du contenant externe (12) ou du contenant interne (11) est fabriqué translucide.

7. Contenant de collecte de sperme (1) selon l'une quelconque des revendications 1 à 6, dans lequel la partie de récupération (15c) comprend un orifice de récupération (15d) ouvert au niveau de la partie inférieure (15b) de la partie de stockage (15) et un film (15e) attaché à l'orifice de récupération (15d).

8. Contenant de collecte de sperme (1) selon l'une quelconque des revendications 1 à 6, dans lequel la partie de récupération (15c) est formée d'une portion fine fournie au niveau d'une partie de la partie inférieure (15b) de la partie de stockage (15).
